# EUROPEAN PATENT APPLICATION

(11) **EP 4 818 336 A1**
(43) Date of publication of application: **07.10.2026**
(21) Application number: 25168688.7
(22) Date of filing: 04.04.2025
(51) Int. Cl.: C12Q 1/689

(54) **METHOD FOR DIAGNOSIS, PROGNOSIS, AND THERAPY MONITORING OF TUBERCULOSIS INFECTION**

(71) Applicant: Fraunhofer-Gesellschaft zur Förderung der angewandten Forschung e.V., 80686 München (DE); Klinikum der Universität München Anstalt des öffentlichen Rechts, 81377 München (DE)
(72) Inventor: Held, Kathrin, 80799 München (DE); Ahmed, Mohamed, 80802 München (DE); Geldmacher, Christof, 80802 München (DE); von Both, Ulrich, 81377 München (DE); Heinrich, Norbert, 80802 München (DE); Höelscher, Michael, 80802 München (DE)
(74) Representative: Kuttenkeuler, David

(57) **Abstract**

A method of diagnosis, prognosis, stratification and/or monitoring of a Mycobacterium tuberculosis (Mtb) infection is provided. The method comprises determining the mRNA expression level of AIM2, GBP1, FCGR1B, CD274, SMARCD3 and FLVCR2 in a blood sample of an individual.

## Description

### FIELD OF THE INVENTION

The invention is based on method of diagnosis, prognosis, stratification and/or monitoring of an infectious disease, especially a Mycobacterium tuberculosis (Mtb) infection. This invention further relates to a diagnostic kit for performing a method according to the present invention, as well as use of a nucleic acid probe or nucleic acid primer in a diagnosis, prognosis, stratification and/or monitoring of an infectious disease, especially a Mtb infection.

### DESCRIPTION

Tuberculosis (TB) remains a formidable global public health challenge, with millions of new cases reported each year. To combat this problem, it requires not only effective treatments, but also accurate and rapid diagnostic tools of both active and latent forms of *Mycobacterium tuberculosis* (M.tb or Mtb) infection, that can perform as reliable indicators of treatment success and TB therapy monitoring.

Existing diagnostic procedures for active tuberculosis disease are based on pathogen detection in difficult-to-obtain patient samples, such as sputum for pulmonary TB or biopsies for extrapulmonary TB. For example, the procedures recommended by the World Health Organisation (WHO) for monitoring TB treatment and diagnosis are based on the detection of M.tb in sputum, combined with the clinical assessment of the medical care provider.

In resource-limited settings, the majority of cases of pulmonary tuberculosis are diagnosed by sputum smear microscopy. This method can be performed with relatively simple technical means and is therefore well suited for regions with resource-limited environments with high TB prevalence. However, smear microscopy has a suboptimal sensitivity and, like all the other methods mentioned above, is based on patient samples that are often difficult to obtain. Children and people living with the human immunodeficiency virus (HIV) in particular have difficulties providing high-quality sputum samples.

In addition, between 15% and 25% of all tuberculosis cases are extrapulmonary. The diagnosis of extrapulmonary TB is often overlooked by sputum evaluation alone and requires invasive sampling, such as CSF or samples of lymph nodes or other tissues.

Pathogen detection by microbiological culture of M.tb is further complicated by its slow growth in culture media, which leads to processing times of up to two weeks or longer. Rapid action and treatment decisions are therefore difficult with this method. This is particularly problematic with low bacterial density in the sputum, such as after the start of treatment or with paucibacillary forms of tuberculosis, which leads to lower sensitivity and a higher contamination rate. In addition, the culture of M.tb requires specialised laboratories suitable for handling biohazardous material.

Molecular methods that detect M.tb DNA, such as the Xpert MTB/RIF test from Cepheid, are more sensitive than the microscopic assessment of sputum smears, but cannot be used in most microscopy centres as a continuous and stable power supply is required to operate the GeneXpert instrument. Battery-powered devices, such as those from Molbio Diagnostics, are better suited for this purpose. However, DNA-based methods are prone provide false positive results even long after the tubercle bacilli have died, which makes the diagnosis of recurrent disease more difficult.

Another problem in TB diagnostics is the clear determination of the presence of a latent infection with M.tb. Currently, this is characterised by a persistent immune response to stimulation by M.tb antigens without signs of clinically manifest TB disease. The WHO estimates that about a quarter of the world's population is infected with M.tb. A precise test to identify TB infection would increase the likelihood that individuals eligible for preventive TB treatment would benefit from such treatment.

There is currently no gold standard test for the diagnosis of TB infection. The two available test classes, interferon gamma release assays (IGRAs) and the tuberculin skin test (TST), are indirect and require a competent immune response to identify TB-infected individuals. Such tests therefore have low sensitivity, particularly in people with immunodeficiency, such as HIV infection. A positive test result with one of the two methods is not in itself a reliable indicator of the risk of progression to active disease.

The WHO TB Action for the WHO Region Europe emphasizes that to reach the End TB Strategy targets, substantial interventions and the rapid introduction of innovations are necessary. This includes a call to early diagnosis of all forms of TB and universal access to drug-susceptibility testing, including the use of rapid tests aiming to improve treatment success rates. Adherence to treatment is challenging and previous reports have suggested that the standard 6-months regimen for drug-susceptible TB may not be required in all cases of pulmonary TB. Hence, diagnostic tools such as host biomarkers are needed in order to allow implementation of a personalised medicine approach by improved treatment monitoring to define an individual's time point of cure.

There is therefore an urgent need for rapid, easily accessible biomarkers, ideally blood sample biomarkers, for the diagnosis, prognosis and monitoring of TB treatment.

Host transcriptome-based diagnostic tools have emerged as a significant advancement in this field. Analysis of the host transcriptome can reveal critical information about gene regulation in response to TB and its treatment.

Currently, there are some transcriptome-based methods under research for M.tb. For example, the Sweeney_3 (Family 65471803 (US2018291452 AA)) signature is currently being developed by Cepheid, Sunnyvale, CA, USA for their cartridge-based GeneXpert system under the name (Xpert-MTB-HR). The Risk_6 (Family 61600363 (US2019249228 AA)) signature is currently under development at QuantuMDx Group Ltd, Newcastle upon Tyne, UK under the name TB-Host Immune Response Assay (TB-HIRA).). Yet, few signatures have been evaluated for their diagnostic potential as host biomarkers for cure.

Thus, it is an object of the invention to provide a quick method based on easily accessible samples, ideally blood samples, for diagnosing, prognosing, stratification and/or monitoring of Mycobacterium tuberculosis (TB) disease. In particular, the present invention provides a method for distinguishing between an active TB disease and a latent TB infection, as well as for prediction of TB relapses.

### BRIEF DESCRIPTION OF THE INVENTION

Generally, and by way of brief description, the main aspects of the present invention can be described as follows:

In **a first aspect,** the invention pertains a method for **the diagnosis, prognosis, stratification and/or monitoring of an infectious disease,** in a subject, comprising the steps of:
(a) Providing a biological sample from the subject,
(b) Determining the level of at least two or more biomarker selected from the group consisting of AIM2, GBP1, FCGR1B, CD274, SMARCD3, and FLVCR2, in the biological sample,
wherein a differential level of at least one biomarker(s) in the biological sample from the subject as determined in step (b) compared to a healthy control or reference value is indicative for the presence of the infectious disease in the subject.

In **a second aspect,** the invention pertains a diagnostic kit for performing a method according to the present invention.

In **a third aspect,** the invention pertains to use of a nucleic acid probe or nucleic acid primer in a diagnostic, prognosis, stratification and/or monitoring of an infectious disease, wherein such nucleic acid primer hybridizes to a transcript of at least one biomarker selected from the group consisting of IM2, GBP1, FCGR1B, CD274, SMARCD3, and FLVCR2.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the object of the present invention is solved by the provision of a method for **the diagnosis, prognosis, stratification and/or monitoring of an infectious disease,** in a subject, comprising the steps of:
(a) Providing a biological sample from the subject,
(b) Determining the level of at least two or more biomarker(s) selected from the group consisting of AIM2, GBP1, FCGR1B, CD274, SMARCD3, and FLVCR2, in the biological sample,
wherein a differential level of at least one biomarker(s) in the biological sample from the subject as determined in step (b) compared to a healthy control or reference value is indicative for the presence of the infectious disease in the subject.

The present invention is therefore advantageous, in that it provides a concise, full blood sample based biomarker signature for the diagnosis, prognosis and treatment monitoring of TB using a semi-quantitative real-time PCR (qPCR), which fulfils the two basic requirements of the WHO-target product profile for triage or monitoring tests to identify subjects with suspected TB. Specifically, the method for diagnosis, prognosis, stratification and/or monitoring of the present invention has a test sensitivity of at least 85 %, preferably at least 90%, and a test specificity of at least 65%, preferably at least 70%.

A "diagnosis" or the term "diagnostic" in context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "prognosis" refers to a forecast as to the probable outcome of the disease as well as the prospect of recovery from the disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually, prognosis is provided as the time of progression free survival or overall survival. In particular, prognosis may refer to a forecast for disease relapse. Disease relapse may refer to the worsening or deterioration of symptoms, or recurrence of an infection after a period of improvement of symptoms or a negative diagnosis of, for example, an infection.

The term "stratification" for the purposes of this invention refers to the advantage that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof.

The term "monitoring a therapy" or "monitoring a disease" means for the purpose of the present invention to observe disease progression in a subject, preferably a subject who receives a therapy. In other words, the subject during the therapy is regularly monitored for the effect of the applied therapy, which allows the medical practitioner to estimate at an early stage during the therapy whether the prescribed treatment is effective or not, and therefore to adjust the treatment regime accordingly.

As used herein, the term "subject" or "patient" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject. In a particularly preferred embodiment, the subject is a mammal, such as a mouse, a rat, a guinea pig, a rabbit, a cat, a dog, a monkey, or a human, preferably a human, such as a human patient.

The term "biological sample" as used herein refers to a sample that was obtained and may be assayed for any one of the biomarkers as disclosed with the present invention, or their gene expression. The biological sample can include a biological fluid (e.g., blood, cerebrospinal fluid, urine, plasma, serum), tissue biopsy, and the like.

A "biomarker" or "marker" in the context of the present invention refers to one or a group of organic biomolecule (s), particularly a polypeptide, a gene, and/or mRNA, which is differentially present in a sample taken from subjects having a certain condition as compared to a comparable sample taken from subjects who do not have said condition. For example, a marker can be a polypeptide or polysaccharide (having a particular apparent molecular weight) which is present at an elevated or decreased level in samples of patients suffering from a pulmonary dysfunction compared to samples of patients with a negative diagnosis.

In this regard, in an embodiment of the present invention, the method according to the invention comprises at least two or more biomarker selected from a group of biomarkers. In a particularly preferred embodiment, the group according to the present invention consists of AIM2, GBP1, FCGR1B, CD274, SMARCD3, and FLVCR2. Details of the biomarkers are given below in Table 1.

**Table 1 lists biomarkers and exemplary control-markers of the present invention.**

| **Gene Name** | **NCBI Gene ID*:** |
|---|---|
| AIM2 (absent in melanoma 2) | 9447 |
| GBP1 (guanylate binding protein 1) | 2633 |
| FCGR1B (Fc gamma receptor Ib) | 2210 |
| CD274 (cluster of differentiation 274) | 29126 |
| SMARCD3 (SWI/SNF related BAF chromatin remodeling complex subunit D3) | 6604 |
| FLVCR2 (FLVCR choline and putative heme transporter 2) | 55640 |
| B2M (beta-2-microglobulin) | 567 |
| HPRT1 (hypoxanthine phosphoribosyltransferase) | 3251 |
| VIM (vimentin) | 7431 |

| | |
|---|---|
| *Gene information was retrieved from the NCBI Gene database (https://www.ncbi.nlm.nih.gov/gene), accessed on April 4, 2025." | |

In the context of the present invention, the above listed biomarkers may refer to the nucleic acid sequence of the gene or pseudogene, their respective transcribed product, in particular RNA products, or their respective peptide products, such as proteins.

In an alternative embodiment, the biomarkers may further comprise molecules whose level undergo changes during the treatment of the infectious disease.

The concise group of biomarkers to be analysed in this method renders it a particular advantage of short testing time. The method therefore may be performed within a short period of time, preferably within one day, for example within 12 hours or 10 hours or more preferably 8 hours.

In the context of the present invention, the term "level", such as in "level of a biomarker" refers to the absolute or relative amount and/or quantity, which can be measured for example by concentration, the number or the volume. In this sense, a "differential level" would refer to a difference, for example a statistically significant increase and/or a decrease in the absolute or relative amount and/or quantity.

Furthermore, in the context of the present invention, the term "presence" may refer to an increase in the level of a molecule, such as a microbial particle, a protein and/or an mRNA, or a positive readout from for example a measurement. Furthermore, "presence", especially the term "presence of an infectious disease" may refer to manifestation of a clinical symptom in a subject.

Conversely, the term "absence" may refer to a decrease in the level of a molecule, such as a microbial particle, a protein and/or an mRNA, or a negative readout from for example a measurement. Furthermore, "absence", especially the term "absence of an infectious disease" may refer to manifestation of a clinical symptom in a subject.

In a related embodiment of the present invention, the step (b) comprises determining the level of at least two or more of the biomarker(s) in the biological sample, preferably determining the level of at least four or more of the biomarker(s) in the biological sample, more preferably determining the level of at least six or more of the biomarker(s) in the biological sample.

In another preferred embodiment, the method according to the present invention comprises further steps of
(c) determining the level of at least one, preferably of at least two, most preferably at least three, control-marker(s) in the biological sample, wherein the control-marker is not a biomarker, preferably wherein the at least one control-marker is a housekeeping control-marker;
(d) normalizing the level of the at least two or more biomarker(s) as determined in (b) with the level of the at least one control-marker as determined in (c).

In the context of this invention, the terms "control", "control-marker", would mean one or more molecules, whose level could serve as a statistical technique for excluding the influence of external or unwanted variables.

Therefore, in an optional embodiment of the present invention, the control-marker of the present invention comprises house-keeping molecule. House-keeping molecules may be understood as any molecule with a relatively constant level across different cells or subject, or constitute molecules important for maintaining basic functions. In particular, house-keeping molecules may comprise house-keeping genes or proteins. Preferably, the control-marker is selected from a group comprising B2M, HPRT1 and VIM (table 1).

In an alternative embodiment of the present invention, step (b) comprises determining the level of at least AIM2, and BGP1, in the biological sample.

In another embodiment of the present invention, step (b) comprises determining the level of at least AIM2, FCGR1B, CD274, and GBP1, in the biological sample.

In another embodiment of the present invention, step (b) comprises determining the level of at least AIM2, FCGR1B, CD274, GBP1, SMARCD3, and FLVCR2, in the biological sample.

According to a preferred embodiment of the present invention, the biological sample of the present invention is a tissue sample or body liquid sample, preferably a blood sample, mostly preferably a whole blood sample.

In this regard, the method of the present invention has the advantage of employing an easy to access sample, such as a blood sample. This would in clinical settings significantly reduce the required effort in sample collection.

In a preferred embodiment of the present invention, the biomarker is an RNA, such as an mRNA transcript, biomarker.

In another preferred embodiment of the present invention, the subject is a human, such as an adult human, for example aged 18 to 90, or is a child or adolescent human for example aged 0 to 17.

In yet another embodiment of the present invention, the infectious disease is a bacterial disease, such as tuberculosis, which is caused by an infection with bacteria of the *mycobacterium tuberculosis* complex. Preferably, the bacteria is Mycobacterium tuberculosis (Mtb). The present invention therefore advantageously provides a method for differential diagnosis between infectious diseases, in particular, for distinguishing between TB from other diseases, such as sarcoidosis.

In an advantageous embodiment, the method is for distinguishing between a first infectious disease caused by an infection with Mycobacterium tuberculosis complex, preferably Mtb and a second disease, preferably wherein the second disease is sarcoidosis. The second disease may comprise any disease or disorder.

In a related embodiment of the present invention, the biomarker is detected using one or more method for the quantification of said transcript or mRNA transcript levels in a sample. The skilled person would be familiar with various techniques in the state of the art for quantification or detection of RNA in a sample. Such techniques may be but are not limited to quantitative Polymerase Chain Reaction (qPCR), Loop mediated Isothermal Amplification (LAMP), next generation sequencing (NGS), probe-based nucleic acid hybridization assays (such as Northern blots and similar techniques).

In a preferred embodiment of the present invention, the biomarker, in particular, a mRNA biomarker, is detected using qPCR.

In a specific embodiment of the present invention, the level of the biomarker detected with qPCR is measured by cycle threshold (Ct). Ct represents the cycle number at which a fluorescence threshold is crossed.

In a further embodiment of the present invention, the level of the biomarker is measured in a normalized value, in particular a normalized Ct value. The skilled person would be aware of different methods of normalization methods, such as normalization with control value, normalization within or across samples or datasets.

In a specific embodiment of the present invention, the level of the biomarker is measured in a normalized level calculated by subtracting the level of a control-marker or a mean, in particular a geometric mean, of two or more control-markers from the level of the biomarker. In particular, wherein the biomarker is an RNA transcript biomarker, the level of biomarker may be measured as a delta cycle threshold (delta Ct or dCt), calculated by subtracting the level, especially the level measured in Ct value, of a control-marker or the geometric mean of two or preferably three, or more control-markers from the level of the biomarker.

The skilled person is aware of various statistical methods for comparing the level of biomarkers using various techniques, with which the differential level of biomarkers may be determined. Said statistical methods may be simple arithmetic manipulation such as subtraction but could also be statistical modelling. In the context of the present invention, a differential level of biomarker would refer to any statistically significant changes of the biomarker level.

In a specific embodiment of the present invention, a differential level of biomarkers may be determined by comparing the level of biomarkers, in particular the normalized biomarker levels, between the subject and a healthy control or reference value. Preferably, a healthy control may be a subject not infected with an infectious disease, in particularly, a subject not or has never been infected with Mycobacterium tuberculosis. Furthermore, a healthy control may also be but is not necessarily a subject not showing any clinical symptoms of an infectious disease. A reference value may be a value or threshold, such as a biomarker level, from one or more subjects not or have never been infected with an infectious disease, and/or from one or more subjects who have completely recovered from an infectious disease. A reference value may further be a predetermined value. The skilled person will understand that numerous methods may be used to select a threshold or reference value for a particular marker or a plurality of markers. In diagnostic aspects, a threshold value may be obtained by performing the assay method on samples obtained from a population of patients infected with a certain microbe.

Additionally, thresholds may be established by obtaining an earlier marker result from the same patient, to which later results may be compared. In some aspects, the individuals act as their own "control group." In markers that increase with disease severity or prognostic risk, an increase over time in the same patient can indicate a worsening of disease or a failure of a treatment regimen, while a decrease over time can indicate remission of disease or success of a treatment regimen.

In another preferred embodiment of the present invention, a differential level of biomarkers is determined by comparing the level of one biomarker or the medium level of two or more biomarkers with the healthy control or reference value.

In another preferred embodiment, the method according to the present invention is for the monitoring of a therapy, in particular therapy success, in the subject.

In yet another preferred embodiment, the method according to the present invention is for detecting a latent or active infection causing the infectious disease, preferably is a tuberculosis infection or is a clinical tuberculosis. In the context of the present invention, the phrase "tuberculosis infection" is interchangeable with "latent tuberculosis infection", and the phrase "clinical tuberculosis disease" is interchangeable with "active tuberculosis infection". In the context of the present invention, an active infection refers to a condition where the infected subject displays symptoms such as fever, fatigue and coughing. A latent infection would in turn refer to a condition where the infected subject does not display any clinical symptoms despite the presence of for example a pathogen. It should be noted that with a subject with latent infection may still exhibit immunological reactions.

In a particularly preferred embodiment of the present invention, the at least one biomarker is a panel of AIM2, FCGR1B, CD274, GBP1, SMARCD3, and FLVCR2.

In another embodiment, the method according to the present invention is a screening method. Screening method may be understood in this context as method for the identification of subjects with a certain criterion. In particular, the screening method of the present invention may be methods for the identification of subjects in need of or suitable for therapy, for example a therapy against an infectious disease.

In a preferred embodiment of the present method, the subject is a human.

In a preferred embodiment the method of the herein disclosed invention is performed in *vitro* or *ex vivo.* Since the herein described diagnostic methods are non-invasive the term "providing a biological" sample shall preferably not be interpreted to include a surgical procedure conducted at the subject.

In a second aspect, the object of the present invention is solved by the provision of a diagnostic kit for performing a method according to the present invention.

For example, the kits can be used to detect any one or any combination of biomarkers described above, which biomarkers are differentially present in samples of a patient with the infectious disease compared to a control subject. The kits of the invention have many applications. For example, the kits can be used to differentiate whether a subject has the infectious disease, or has a negative diagnosis, thus aiding a diagnosis. In another example, the kits can be used to identify whether a subject is in need of a treatment for the infectious disease.

In a preferred embodiment, the diagnostic kit is used for therapy monitoring, such as monitoring the progression of the infectious disease in response to a therapy. In a related embodiment, the diagnostic kit may be used for prognosis as well.

In a preferred embodiment, the diagnostic kit according to the present invention may comprise one or more mean(s) for the detection of the at least one biomarker(s), preferably wherein the mean(s) are selected from a nucleic acid primer and/or probe or is an antibody.

A skilled person would understand that based on context, the term "mean" could either refer to an arithmetic average or refer to an action or a method by which a result is achieved.

Optionally, the kit can further comprise instructions for suitable operational parameters in the form of a label or a separate insert. For example, the kit may have standard instructions informing a consumer how to determine the level of more than one biomarkers using the component contained therein.

In either embodiment, the kit may optionally comprise a standard or control information so that the test sample can be compared with the control information standard to determine if the test amount of a marker detected in a sample is a diagnostic amount consistent with a diagnosis of an infectious disease in the subject.

In **a third aspect,** the object of the present invention is solved by use of a nucleic acid probe or nucleic acid primer in a diagnostic, prognosis, stratification and/or monitoring of an infectious disease, wherein such nucleic acid primer hybridizes to a transcript of at least one biomarker selected from the group consisting of AIM2, GBP1, FCGR1B, CD274, SMARCD3, and FLVCR2, and optionally wherein such nucleic acid primer hybridizes to a transcript of one or more control-marker, preferably wherein the control-marker is selected from the group comprising B2M, HPRT1, and VIM.

A nucleic acid probe according to the present invention may be a nucleic acid molecule capable of binding to the biomarker and/or control-marker, especially an RNA biomarker and/or control-marker according to the present invention. A nucleic acid primer according to the present invention may be a nucleic acid molecule capable of binding to the biomarker and/or control-marker, especially for the purpose of sequence amplification and subsequent detection or quantification of the biomarker and/or control-marker.

In a specific embodiment of the present invention, the nucleic acid primers comprise sequences according to SEQ ID NO: 1 to 18.

In a preferred embodiment, the use is the method for the diagnosis, prognosis, stratification and/or monitoring of an infectious disease according to the present invention.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1. A method for the diagnosis, prognosis, stratification and/or monitoring of an infectious disease, in a subject, comprising the steps of:
   (c) Providing a biological sample from the subject,
   (d) Determining the level of at least two or more biomarker(s) selected from the group consisting of AIM2, GBP1, FCGR1B, CD274, SMARCD3, and FLVCR2, in the biological sample,
   wherein a differential level of at least one biomarker(s) in the biological sample from the subject as determined in step (b) compared to a healthy control or reference value is indicative for the presence of the infectious disease in the subject.
Item 2. The method of item 1, wherein step (b) comprises determining the level of at least two or more of the biomarker(s) in the biological sample, preferably determining the level of at least four or more of the biomarker(s) in the biological sample, more preferably determining the level of at least six or more of the biomarker(s) in the biological sample.
Item 3. The method of item 1 or 2, wherein the method comprises further steps
   (e) of determining the level of at least one, preferably of at least two, most preferably at least three, control-marker(s) in the biological sample, wherein the control-marker is not a biomarker, preferably wherein the at least one control-marker is a housekeeping control-marker, more preferably wherein the at least one control-marker is selected from a group comprising B2M, HPRT1 and VIM;
   (f) normalizing the level of the at least two or more biomarker(s) as determined in (b) with the level of the at least one control-marker as determined in (c).
Item 4. The method of any one of items 1 to 3, wherein step (b) comprises determining the level of at least AIM2, and GBP1, in the biological sample.
Item 5. The method of any one of items 1 to 4, wherein step (b) comprises determining the level of at least AIM2, FCGR1B, CD274, and GBP1, in the biological sample.
Item 6. The method of any one of items 1 to 5, wherein step (b) comprises determining the level of at least AIM2, FCGR1B, CD274, GBP1, SMARCD3, and FLVCR2, in the biological sample.
Item 7. The method of any one of items 1 to 6, wherein the biological sample is a tissue sample or body liquid sample, preferably a blood sample, most preferably a whole blood sample.
Item 8. The method of any one of any of items 1 to 7, wherein the biomarker is an RNA, such as an mRNA transcript, biomarker.
Item 9. The method of any one of any of items 1 to 8, wherein the subject is a human, such as an adult human, for example aged 18 to 90, or is a child or adolescent human for example aged 0 to 17.
Item 10. The method of any one of any of items 1 to 9, wherein the infectious disease is a bacterial disease, such as tuberculosis, for example caused by an infection with bacteria of the Mycobacterium tuberculosis complex, preferably caused by *Mycobacterium tuberculosis .*
Item 11. The method of any one of the preceding items, wherein the biomarker is detected using one or more method for the quantification of transcript levels in a sample, such as quantitative Polymerase Chain Reaction (qPCR), Loop-mediated Isothermal Amplification (LAMP), next generation sequencing (NGS), probe-based nucleic acid hybridization assays (such as Northern Blots and similar technologies).
Item 12. The method of any one of the preceding items, wherein the method is for the monitoring of a therapy, in particular therapy success, in the subject, and/or wherein the method is a screening method.
Item 13. The method of any one of the preceding items, wherein the method is for distinguishing between a first infectious disease caused by an infection with Mycobacterium tuberculosis and a second disease, preferably wherein the second disease is sarcoidosis,
and/or wherein the method is for the detecting a latent or active infection causing the infectious disease, preferably is a tuberculosis infection (latent tuberculosis infection) or is a clinical tuberculosis disease (active tuberculosis infection), preferably wherein the at least one biomarker is a panel of AIM2, FCGR1B, CD274, GBP1, SMARCD3, and FLVCR2.
Item 14. A diagnostic kit for performing a method of any one of the preceding items, preferably comprising one or more means for the detection of the at least one biomarker(s), preferably wherein the means are selected from a nucleic acid primer and/or probe, or is an antibody.
Item 15. Use of a nucleic acid probe or nucleic acid primer in a diagnosis, prognosis, stratification and/or monitoring of an infectious disease, wherein such nucleic acid probe or nucleic acid primer hybridizes to a transcript of at least one biomarker selected from the group consisting of AIM2, GBP1, FCGR1B, CD274, SMARCD3, and FLVCR2, and optionally wherein the nucleic acid primer hybridizes to a transcript of one or more control-marker, preferably wherein the control-marker is selected from the group comprising B2M, HPRT1, and VIM, preferably wherein the use is in a method of any one of items 1 to 13.

### BRIEF DESCRIPTION OF THE FIGURES AND SEQUENCES

The figures show:

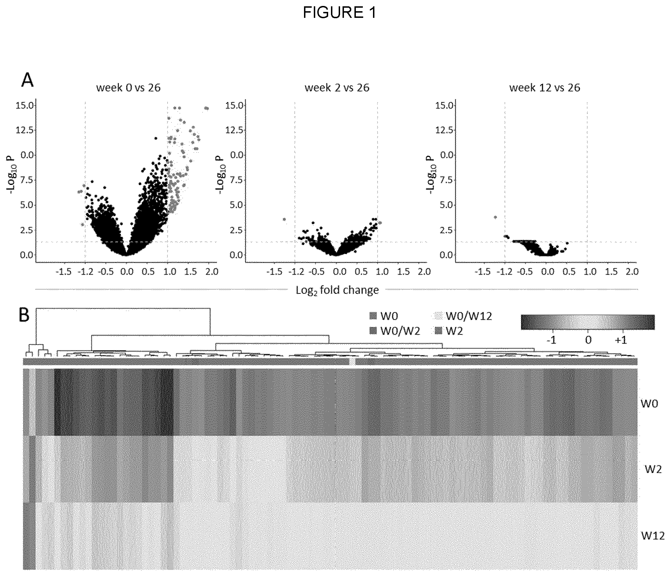

**Figure 1** **shows:** Transcriptional changes during TB treatment. Volcano plots illustrating global transcriptional changes from baseline (W0), 2 weeks and 12 weeks into treatment to end of treatment (EOT, W26) **(A).** Each dot represents a gene, with log2 fold change at the indicated time point vs. week 26 plotted against the -log10 of the p-value. The cut-offs for significantly regulated genes (highlighted in red), a p-value < 0.05 and fold change of >|1|, are indicated by dashed lines. Heat map depicting color-coded expression levels of the 98 significantly differentially expressed genes from baseline to 12 weeks into treatment vs. EOT **(B).** The dendrogram was generated using Euclidean distances and Ward's clustering. Genes exclusive to WO and W2 are denoted in blue and red, respectively, while those present in both WO and W2 and WO and W12 are depicted in purple and cyan, respectively.

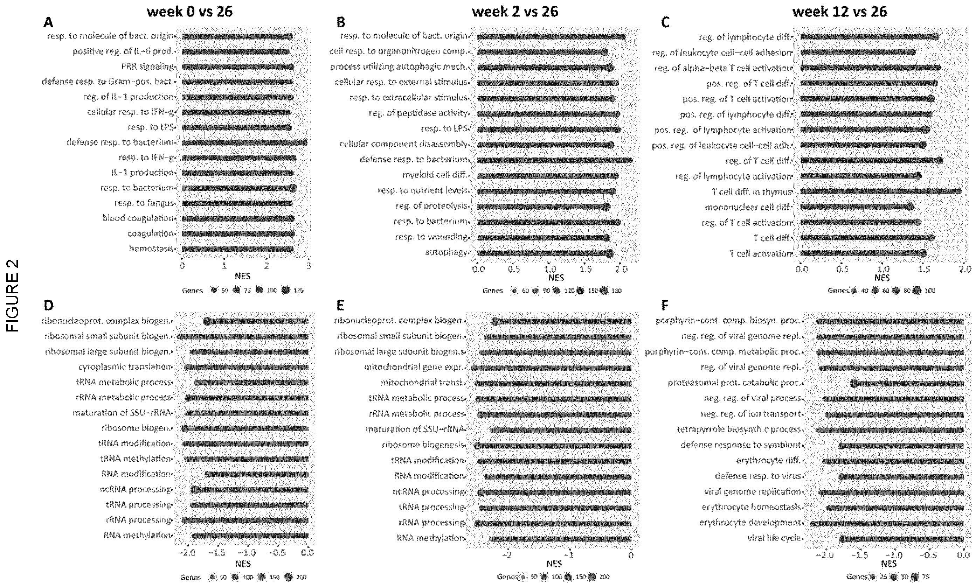

**Figure 2****:** shows GO Term enrichment analysis over TB treatment course. GO Term enrichment analysis of up or down regulated genes for each time point relative to the end of treatment at week 26. For each comparison, the 15 most significant up (A-C) or down (D-F) regulated pathways are depicted. The length of the stubs shows normalised enrichment scores and dot sizes represent the number of genes that contribute to each term's enrichment.

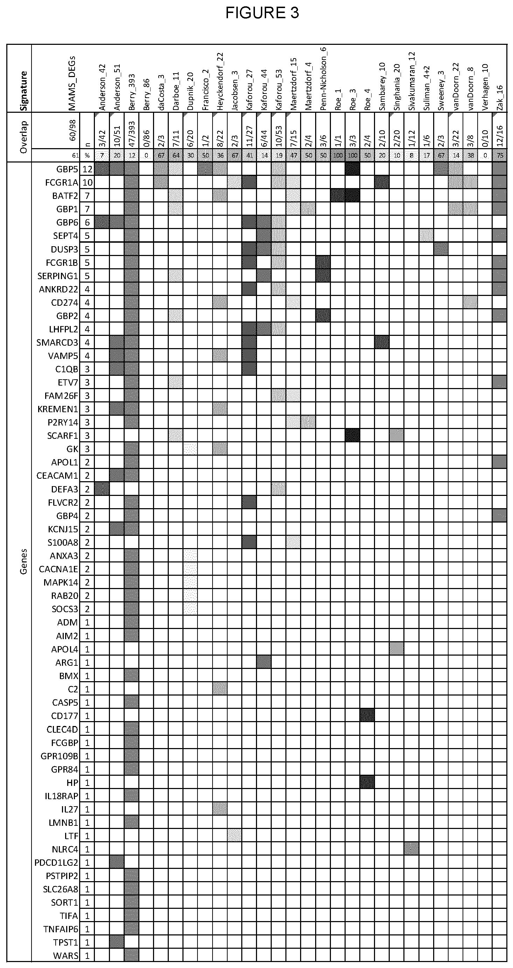

**Figure 3****:** shows overlap of the 98 MAMS DEGs with published signatures. Comparison between genes identified as differentially expressed over the treatment course in the MAMS cohort and those present in published signatures. Each column represents a distinct published gene signature for diagnosing clinical TB, predicting the progression from latent tuberculosis infection, assessing *Mtb* sputum load, or evaluating treatment response and outcome. Each row corresponds to a single DEG identified by RNA sequencing in the MAMS training cohort (MAMS_DEGs). Only genes occurring in other signatures are listed (60 out of 98; 61%). Signatures are denoted by a combination of the first author's name from the respective publication and the number of constituent genes. The degree of overlap between the MAMS_DEGs and each signature is indicated by the number of shared genes out of the total signature genes, along with the corresponding percentage. Genes are arranged based on their frequency of occurrence in published signatures. Each signature is denoted by an individual colour.

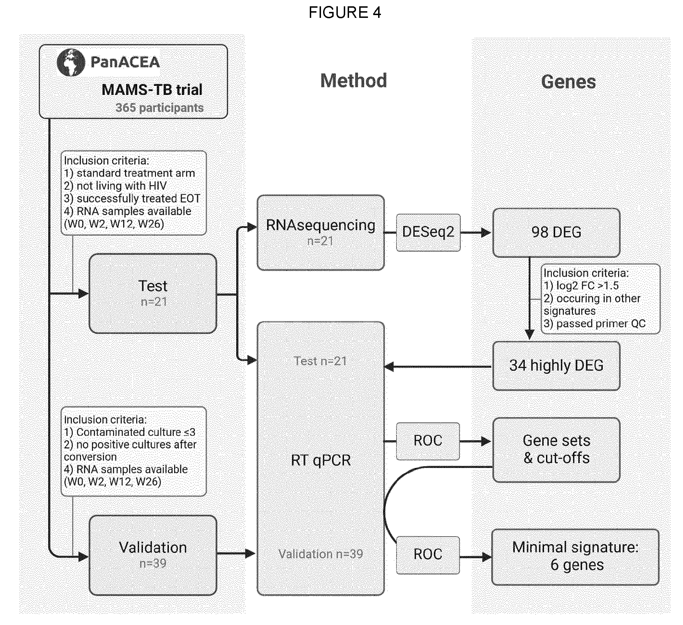

**Figure 4****:** shows STARD Diagram Illustrating Participant Selection and Analysis. Participants from the MAMS cohort underwent selection for RNA sequencing and subsequent qPCR validation sets based on listed criteria. Genes exhibiting significant differential expression via RNA sequencing in the training set were further investigated through qPCR analysis across both cohorts. Subsequently, genes demonstrating robust discriminatory potential, as identified through ROC analysis in the test set, were validated via qPCR in the validation set, culminating in the identification of a minimal size 6-gene signature. EOT = end of treatment; W = week; ROC = receiver operating characteristic; DEG = differentially expressed genes; FC = fold change; QC = quality control.

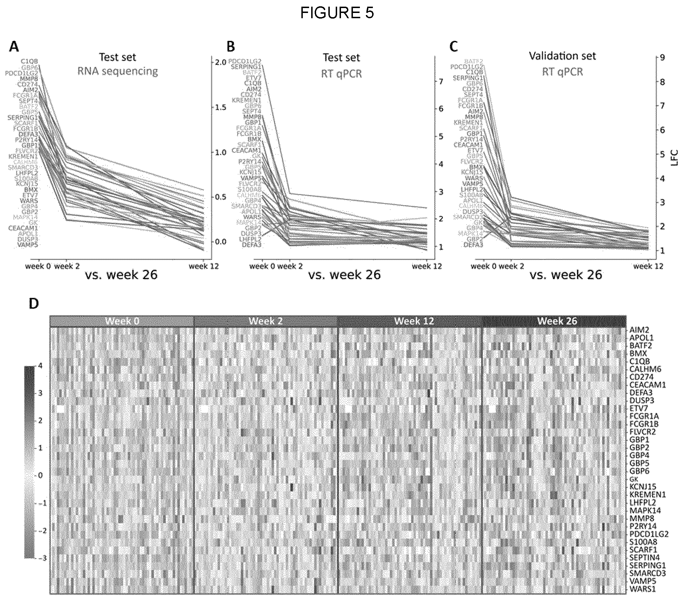

**Figure 5****:** shows Changes in Gene Expression of Potential Signature Genes during TB Treatment. Fold changes in gene expression of all 34 immune-related potential signature genes relative to EOT, determined by RNA sequencing (A), qPCR in the test set (B), and RT-qPCR in the validation set (C). Panel A depicts the log fold change relative to week 26 from the RNA sequencing data for each single gene. Panels B and C depict the median 2^{-ΔΔCt} values calculated with week 26 as the reference for each participant. (D) Heat map illustrating individual dCT values for each MAMS participant at each measured time point from both the training and validation cohorts. Coloured bars denote the weeks of treatment.

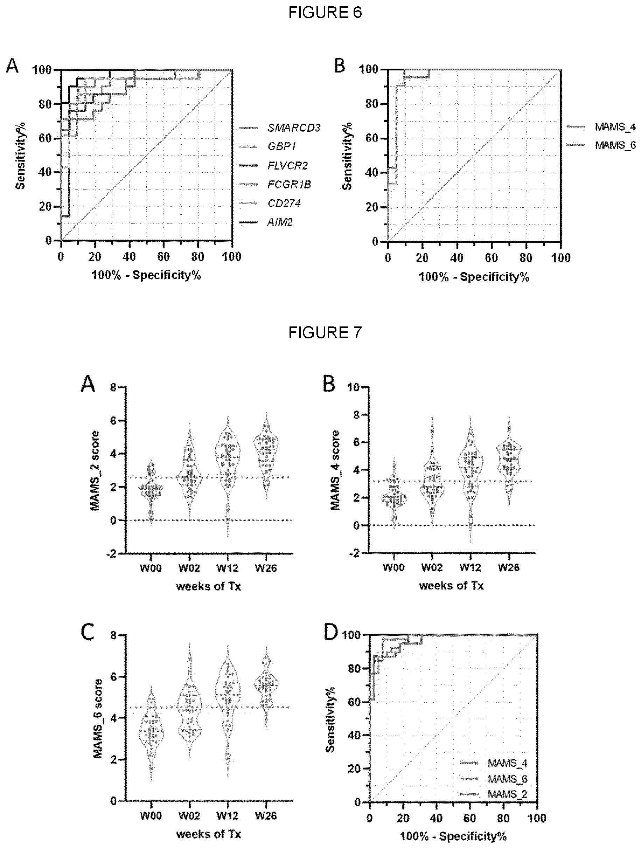

**Figure 6****:** shows ROC curves of the 6 most discriminating genes and both signatures in the training set. ROC curves for discriminating between Tx start and EOT in the training cohort as measured by blood transcript levels of *SMARCD3, GBP1, FLVCR2, FCGR1B,* CD274, and *AIM2* (A) and combination signatures MAMS_4 and MAMS_6 (B). Blood transcript levels of each gene are stated as dCT values and calculated signature scores, respectively. AUC and 95% CI as well as optimal cut-offs reaching 90% sensitivity are listed in Table 2.
**Figure 7****:** shows transcriptomic signatures accurately identify cured TB patients. Treatment monitoring using combination signatures MAMS_2 (A), MAMS_4 (B) and MAMS_6 (C) in the MAMS validation cohort. Blood transcript levels are stated as calculated signature scores for each participant using the median dCT of all signature genes for each analysed time point (weeks of Treatment =Tx). Violin plots depict density of data points per time point. Median and quartiles are indicated by dotted black lines for each violin. Red dotted lines indicate cut-offs as determined in the training cohort for at least 90% sensitivity and 70 % specificity for cure at EOT (MAMS_2: 2,570, MAMS_4: 3,180, MAMS_6: 4,526 (Table 2)). ROC curves for discriminating between Tx start and EOT in the validation cohort for combination signatures MAMS_2, MAMS_4 and MAMS_6 (D).

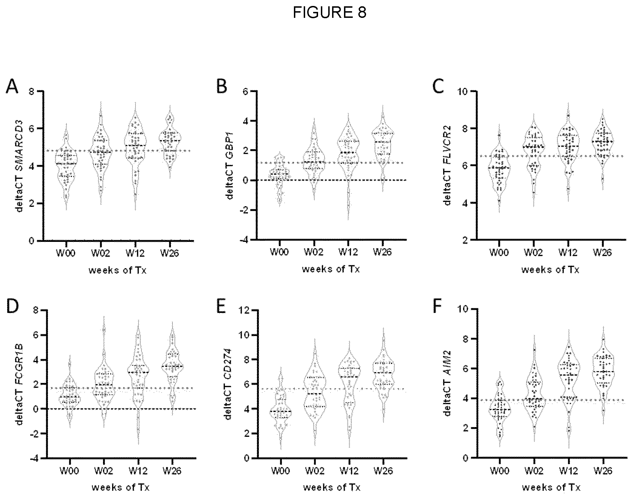

**Figure 8****:** shows Expression of 6 Signature genes over treatment course. Treatment monitoring using transcript levels of genes with the highest discriminatory power in the training set: *SMARCD3* (A), *GBP1* (B), *FLVCR2* (C), *FCGR1B* (D), CD274 (E), and *AIM2* (F) in the MAMS validation cohort. Blood transcript levels of each gene are stated as dCT values for each participant and measured time point after treatment (Tx) initiation. Violin plots depict the density of data points per time point. Median and quartiles are indicated by dashed black lines for each violin. Red dashed lines indicate cut-off as determined in the training cohort for at least 90% sensitivity and 70 % specificity for cure at EOT (Table 2).

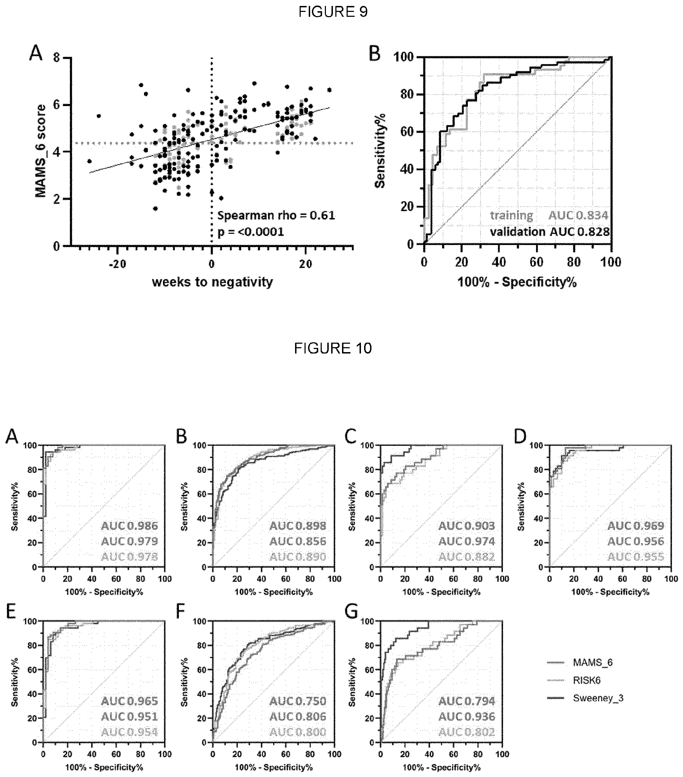

**Figure 9****:** shows correlation of MAMS_6 Signature with Time Interval around Stable Culture Conversion. MAMS_6 score for each individual participant measurement is plotted against weeks preceding and succeeding stable culture conversion in the training (grey dots) and validation (black dots) cohort (A). The red dashed line indicates the optimal cut-off at 4.37, determined in the training cohort (grey) for achieving at least 90% sensitivity. Spearman rank correlation resulted in and rho of 0.6085 with a p-value of <0.0001. The trendline is shown. ROC curves illustrating the discrimination between before and after stable culture conversion using the MAMS_6 signature in the training and validation cohorts (B). Employing the cut-off depicted in (A) yields a sensitivity of 86.3% and specificity of 62.65% in the validation cohort.
**Figure 10****:** shows diagnostic performance of the MAMS_6 Signature in five independent published cohorts ROC for 3 parsimonious signatures based on RNA sequencing data of the Leicester cohort (A,E) microarray data of two African cohorts (B,F) and an Indian paediatric cohort (C,G) as well as an extrapulmonary TB cohort (D). Comparisons are as follows: A&D: for healthy controls vs TB disease; B,C&E: TB infection (TBI) vs TB disease; F&G: other respiratory diseases (OD) vs TB disease (D).

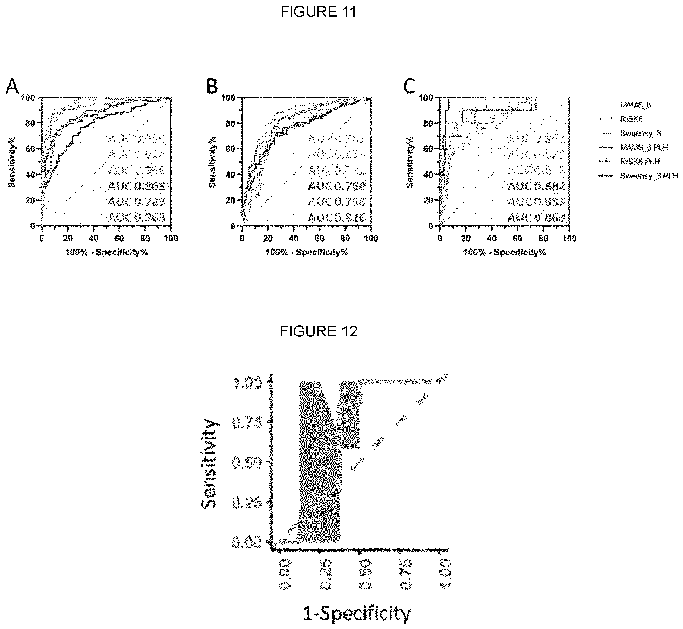

**Figure 11****:** shows diagnostic performance of three transcriptomic signatures in in PLH. ROC for 3 parsimonious signatures based on microarray data of two African cohorts (A,B) and an Indian paediatric cohort (C). ROC depicting distinction of TBI from TB disease (A) and OD vs TB (B,C) split by HIV status.
**Figure 12****:** shows Prediction of relapse at EOT using the MAMS_6 signature in the MAMS cohort. ROC depicting the distinction of patients with recurrent disease from those without.

The sequences show:
SEQ ID NO. 1 to 2 shows forward and reverse primer of AIM2, respectively:
   SEQ ID NO. 1: GCATCTGCAGCCATCAGAAA
   SEQ ID NO. 2: GCCACCATCTGTTTCTGTTCA
SEQ ID NO. 3 to 4 shows forward and reverse primer of GBP1, respectively:
   SEQ ID NO. 3: ACACCAGATGAGTACCTGACA
   SEQ ID NO. 4: ACAGAGTCTGGGCAGGTTAA
SEQ ID NO. 5 to 6 shows forward and reverse primer of FCGR1B, respectively:
   SEQ ID NO. 5: CAGATTTCACTACTCCCACCAG
   SEQ ID NO. 6: CCAAAGGAGCAGAGTTGTCA
SEQ ID NO. 7 to 8 shows forward and reverse primer of CD274, respectively:
   SEQ ID NO. 7: ACCAGCCGCGCTTCTGT
   SEQ ID NO. 8: TCAGCAAATGCCAGTAGGTCATGAAT
SEQ ID NO. 9 to 10 shows forward and reverse primer of SMARCD3, respectively:
   SEQ ID NO. 9: GGACTCCCATGACAAGGAATACA
   SEQ ID NO. 10: AAAACTTCAGCCGGGGACAA
SEQ ID NO. 11 to 12 shows forward and reverse primer of FLVCR2, respectively:
   SEQ ID NO. 11: GTTCATCACTGCTGGCACAA
   SEQ ID NO. 12: TACGTGAGCTCCACAGCAA
SEQ ID NO. 13 to 14 shows forward and reverse primer of B2M, respectively:
   SEQ ID NO. 13: TTAGCTGTGCTCGCGCTAC
   SEQ ID NO. 14: CTCTGCTGGATGACGTGAGTAA
SEQ ID NO. 15 to 16 shows forward and reverse primer of VIM, respectively:
   SEQ ID NO. 15: CCTGTGAAGTGGATGCCCTTA
   SEQ ID NO. 16: CAACGGCAAAGTTCTCTTCCA
SEQ ID NO. 17 to 18 shows forward and reverse primer of HPRT1, respectively:
   SEQ ID NO. 17: GCTTTCCTTGGTCAGGCAGTA
   SEQ ID NO. 18: ACTTCGTGGGGTCCTTTTCAC

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Treatment initiation induces rapid changes in gene expression profiles

Whole blood genome-wide transcriptional profiles of pulmonary tuberculosis (TB) patients were assessed via RNA sequencing of samples from baseline (week(W) 0) and weeks(W) 2, 12, and 26 post-TB treatment initiation. The ratios of fold changes were determined for three pairwise comparisons (WO vs. W26, W2 vs. W26 and W12 vs. W26; Figure 1A). Genes meeting the criteria of a p-value < 0.05 and a log fold change > |1| were considered significantly differentially expressed. The largest proportion of significantly differentially expressed genes (DEG) was observed in the WO vs. W26 comparison, with 97 genes identified, compared to 3 and 1 genes in the W2 and W12 vs. W26 comparisons, respectively (Figure 1A). Notably, as TB treatment continued, a general trend of reduction of gene expression was observed (Figure 1B), with 93 out of the 97 genes upregulated and only 4 genes downregulated from baseline to end of treatment (EOT). As early as two weeks into treatment, a markedly different gene expression landscape was observed, with only three DEGs (*GBP6, MMP8,* and *USP18*) compared to EOT (Figure 1A-B). By week 12, the overall expression profile largely resembled that of EOT (week 26), with only *IFI27* showing differential expression (Figure 1A-B). Despite being significant at only two time points, IFI27 remained consistently down-regulated throughout treatment. The DEGs were predominantly associated with host response to infection, including neutrophil and leukocyte activation, as well as inflammation (cytokine signalling, neutrophil degranulation, interferon gamma (IFNg) signalling, IL signalling, TLR cascades, antimicrobial peptides).

Interestingly, gene ontology (GO) revealed a dynamic pathway profile throughout treatment (Figure 2). At baseline, pathways related to cellular response to pathogens, type I and II IFN responses, cytokine and chemokine production, leukocyte chemotaxis and function, blood metabolism, and wound healing were upregulated, while RNA metabolism pathways were downregulated. At week 2, similar trends were observed, with additional downregulation of pathways involved in T and B lymphocyte activation and type I IFN responses. By week 12, far fewer enriched gene sets were identified, yet there was a notable upregulation of T-cell processes. Additionally, pathways related to erythrocyte life cycle and cellular response to pathogens, upregulated at week 2, were downregulated at week 12 compared to EOT (Figure 2). The 15 most significant up- or downregulated GO terms are listed in Figure 2.

### Example 2: Selection of potential signature genes

Building upon our observations of transcriptomic changes during treatment, our goal was to pinpoint a minimal gene set capable of discriminating between patients cured from pulmonary TB and those with clinical disease. Using this minimal gene set, we then aimed to develop a RT-qPCR based diagnostic signature easily applicable in primary health care clinics where rapid and accurate diagnosis is crucial for effective patient management and disease control.

In recent years, numerous blood-based gene-expression biomarker signatures derived from host responses to TB have been reported for diagnosing clinical TB, predicting progression from latent tuberculosis infection, assessing *Mtb* sputum load, and evaluating treatment response and outcome across various clinical scenarios. By comparing the 98 DEGs over the treatment course in the MAMS cohort to published signatures (Figure 3), we found that 60 out of the 98 genes (61.22%) occur in other signatures. Among the genes most frequently encountered in these published signatures (n >5), all but *STAT1* are also differentially expressed over the treatment course. Apart from the Roe_1 and Roe_3 signatures, initially discovered for risk of incident TB disease, no signature is fully encapsulated by the 98 treatment DEGs. Notably, most signatures share only a few genes and display considerable divergence. Noteworthy overlaps were observed with the Zak_16 (12/16), Sweeney_3 (2/3), Jacobsen_3 (2/3), and DaCosta_3 (2/3) signatures. No overlapping genes could be identified with the Berry_86, and Verhagen_10 signatures.

Genes for RT-qPCR validation were selected based on the premise that genes occurring consistently in various signatures derived from diverse clinical cohorts that include transcriptomic data from varying geographical areas, age groups, or patients living with comorbidities are bound to be relevant for a diverse setting. Therefore, out of the 98 DEGs over the course of treatment in our own cohort, we selected genes also occurring in other signatures (**Figure 3**) and presenting with high differential expression (fold change >1.5) over the treatment course. Moreover, only genes with primer pairs that passed stringent testing for efficient amplification, linearity, and multiplexing capability, as outlined in Material and Methods, were included in the final selection of potential signature genes. Additionally, we incorporated 3 housekeeping genes (HKG) *(B2M, VIM,* and *HPRT1*) with varying expression ranges for normalisation of RNA input.

### Example 3: RT-qPCR results confirm rapid decline in expression of immune-related genes following treatment initiation

We then assessed the gene expression of the selected 34 potential signature genes in 21 participants from the training cohort and in all 39 participants of the validation cohort by microfluidic RT-qPCR (Figure 4). Overall, apart from DEFA3, gene expression measured by RT-qPCR aligns with RNA sequencing findings, demonstrating a swift decrease in all immune-related signature genes after treatment initiation, particularly evident up to week 2, with less pronounced declines thereafter (Figure 5A-C). Notably, genes exhibiting the most rapid decline from baseline to week 2 in both the training and validation cohorts (Figure 5 B and C), as measured by qPCR, encompass components of the complement cascade (*C1QB* - beta chain polypeptide of serum complement subcomponent C1q; *SERPING1-* C1 inhibitor), proteins involved in IFN and cytokine signalling (*BATF -* nuclear basic leucine zipper protein, belonging to the AP-1/ATF superfamily of transcription factors, *GBP6* - IFN induced Guanylate Binding Protein Family Member 6) as well as in TGF-beta mediated apoptosis (*SEPT4*) and inhibition of T-cell activation and subsequent cytokine production (*CD274* - Programmed Cell Death 1 Ligand 1, *PDCD1LG2* - Programmed Cell Death 1 Ligand 2). Interestingly, the expression of *DEFA3* (Defensin, alpha 3), highly abundant in neutrophil granules, increases over the first two weeks of treatment before it rapidly declines again. Plotting the dCT values for each individual MAMS participant (Figure 5D) across the four measured time points reveals substantial inter-patient variability in gene expression, with an overall decrease observed over the treatment course, most notably following treatment initiation.

### Example 4: Translation of RNA sequencing datasets into a parsimonious RT-qPCR-based signature for cure

Only qPCR assays passing quality control in the dilution series were included. Ct values were calculated with an amplification curve quality threshold of 0.65, derivative linear baseline correction, and automated global Ct thresholding. Delta ct (dCT) values were calculated by subtracting the geometric mean of three housekeeping genes' raw Ct values from the raw Ct of target genes. Housekeeping genes were selected based on different expression ranges (B2M: high, HPRT1: low, VIM: median). Experiments were run in duplicate, and mean values analysed. dCT values with duplicate standard deviation >2 or undetected RNA were excluded. Only samples with no non-template control detection, single melting curve peaks, and <20% failed reactions were analyzed.

To identify genes reliably distinguishing clinical TB from cured individuals, a receiver operating characteristic (ROC) analysis was performed for each gene on the training cohort (Table 2). Genes meeting minimal WHO triage test thresholds (sensitivity >90%, specificity >70%) and an area under the curve (AUC) >0.9 were selected and combined into 4- and 6-gene signatures, starting with top-performing genes. Signature performance was subsequently evaluated on the validation cohort using dCT cut-offs, established in the training set to achieve at least 90% sensitivity and 70% specificity (Table 2). Associations between clinical and demographic covariates and the MAMS_6 signature were examined by Spearman's rank correlation, point biserial, or ANOVA for continuous, dichotomous, and nominal covariates, respectively.

Publicly available RNA sequencing and microarray data from TB cohorts (GSE107994, GSE37250; GSE39941, and GSE83456) were accessed via the GEO. GSE107994 was analysed. Size factors were estimated using default settings, followed by variance stabilizing transformation (vst). Resulting normalized expression values were used as input for signature score calculation. For the microarray-based GSE37250, GSE39941, and GSE83456 probes were collapsed to genes, and averaged values were used for signature score calculation.

In order to identify genes that can discriminate cured from clinical pulmonary TB, we performed ROC analyses for each of the 34 potential signature genes in the training cohort. Adhering to the WHO TPP minimal criteria for triage tests, we established the dCT cut-off for each gene at 90% sensitivity for detecting cure, instead of employing the Youden index. Table 2 provides a comprehensive overview, listing the AUC, p-value, suggested cut-off, as well as sensitivity and specificity in descending order of AUC. Genes meeting the WHO TPP minimal criteria of at least 90% sensitivity and 70% specificity, along with an AUC above 0.9, were integrated into a 4-gene signature, comprising *AIM2, FCGR1B,* CD274, and *GBP1* (MAMS_4). Further, genes with an AUC above 0.9 were consolidated into a 6-gene signature, including *AIM2, FCGR1B, CD274, GBP1,* and additionally *SMARCD3,* and *FLVCR2* (MAMS_6) (Figure 6). For both signatures, a score was calculated using the median dCT of all signature genes measured at each time point.

Subsequently, we tested performance of both signatures, as well as each comprising gene, in the validation cohort using the cut-offs determined in the training set (Table 2, Figure 7). MAMS_4 accurately classified 34 out of 39 participants as cured at EOT and 33 out of 39 as clinical TB at time of diagnosis (W0), resulting in a sensitivity of 87% and a specificity of 85%, missing the WHO TPP minimal criteria by a small margin when applying training set cut-off. Conversely, the MAMS_6 signature correctly classified 38 out of 39 participants as cured at EOT and 36 out of 39 as clinical TB at treatment initiation, achieving a sensitivity of 97% and a specificity of 92%, well within the WHO TPP criteria for a triage test (Figure 7). Moreover, *GBP1* (sensitivity 92.31% and specificity 87.18%) as well as *AIM2* (sensitivity 97.44% and specificity 76.92%) both exhibited sufficient discriminative performance for cure in patients with drugsensitive TB at week 26 (Figure 8). Integrating these two genes into a dual-gene signature yielded a sensitivity of 92.31% and a specificity of 82.05% (Figure 7). This performance surpasses that of the four-gene signature, though it remains inferior to the six-gene signature.

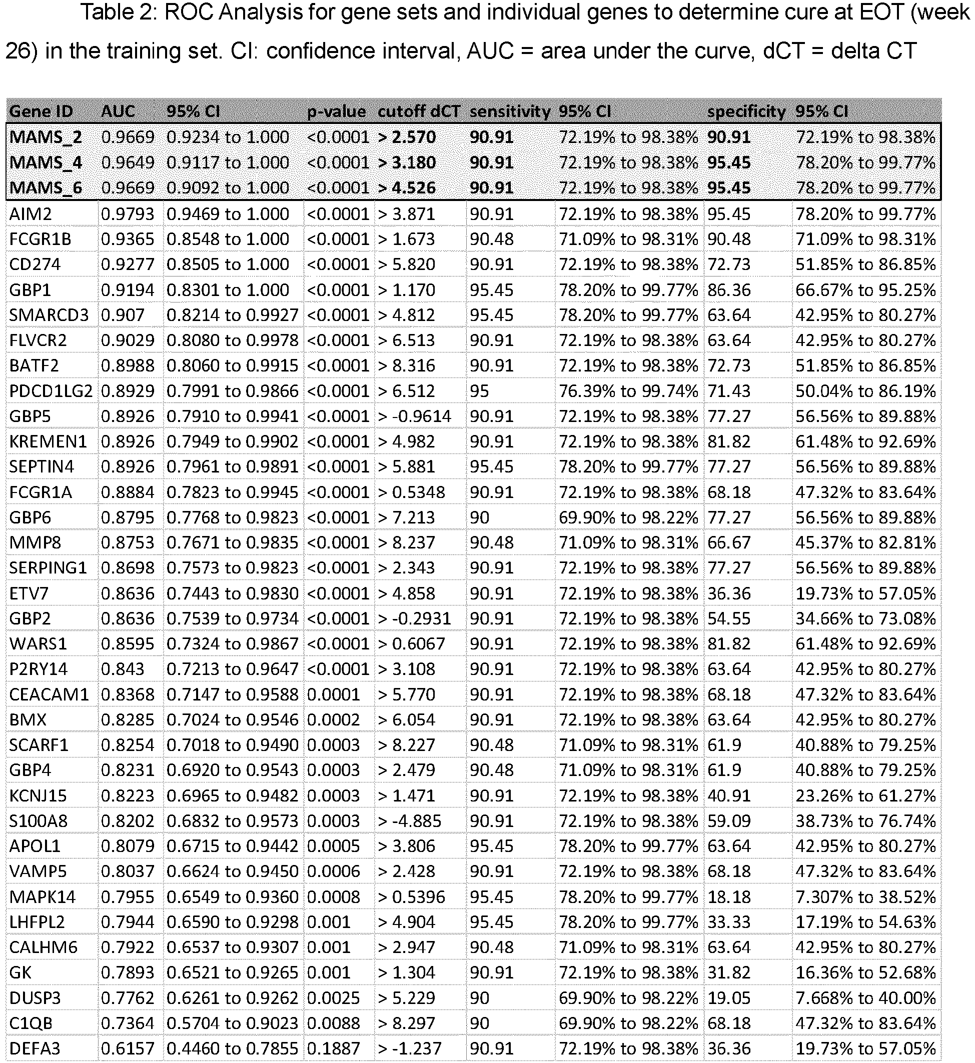

### Example 5: MAMS_6 score is not impacted by treatment regimen, sex or age of the individuals

The universality of the MAMS_6 signature for cure was investigated by examining the relationship between clinical covariates such as sex, age, BMI, HIV status, treatment regimen, site, percent lung involvement as determined by chest x-ray, and number of cavities and the MAMS_6 signature. Regarding HIV status, only one patient in the subset cohort tested positive for HIV, precluding a meaningful comparison. Examination of the signature scores for this participant, revealed that the score values for this participant consistently exceeded the cure threshold. The effect of site was only investigated in participants from sites that had more than 2 participants, leaving only 4 of the 7 MAMS-TB sites.

Within the continuous covariate, strongest association was observed between percentage of lung tissue affected and MAMS_6 score at both treatment initiation (WO0: Spearman's rho: - 0.32; adj. P-value 0.159) and 2 weeks into treatment (WO2: Spearman's rho: -0.39, adj. P-value: 0.0292), yet only reaching statistical significance at WO2. Of the categorical variables, significant associations were only identified between MAMS_6 signature and study site at 2 and 12 weeks into treatment (WO2: F statistic 3.0128; p-value 0.0384; W12: F statistic 3.2001; p-value 0.0309). Additionally, the associations between clinical covariates and the fold change (FC) of MAMS_6 score across timepoints was examined. No statistically significant association between any of the clinical covariates and FC of the MAMS_6 score was observed at any timepoint (data not shown). These analyses collectively suggest that clinical factors do not significantly impact the signature, although the signature score may partially reflect the extent of disease.

### Example 6: MAMS_6 transcriptomic signature correlates with stable culture conversion

An important relationship to explore in biomarker analyses is the correlation between these biomarkers with current diagnostic gold standards such as detection of *Mycobacteria* in sputum by liquid culture. To this end we first assessed whether any transcriptomic changes correlated to a *faster* or *slower* sputum culture conversion. Participants from the test cohort were categorized into two groups based on their *time to stable MGIT culture conversion:* those experiencing fast culture conversion before week 8 (G1, n = 12), and those with slower conversion after week 8 (G2, n = 11). Only subjects for whom the precise time of culture conversion, as defined by the first of two consecutive negative weekly sputum cultures without an intervening positive or contaminated culture in liquid media, could be determined were included in this analysis. Comparisons of fold changes measured by RNA sequencing between W26 and WO were performed separately for G1 and G2.

The MAMS_6 score exhibited a significant correlation to the number of weeks preceding and succeeding stable culture conversion (Spearman's rho = - 0.6085; p-value <0,0001; Figure 9 A). ROC analysis for the detection of stable culture conversion yielded an AUC of 0.83 for the training cohort (Figure 9 B). Applying the cut-off determined for at least 90% sensitivity (MAMS_6 score 4.37) resulted in a specificity in the training cohort of 66.67%. Incidentally, this cut-off is also in line with the Youden's J (0.58). Application of this cut-off to the validation cohort yielded an 86.3% sensitivity and 62.65% specificity (Figure 9 B). Alternatively, utilizing Youden's J (0.54), a sensitivity of 76.71% and specificity of 77.11% were achieved with a cut-off of 4.773 MAMS_6 signature score. Furthermore, associations of the MAMS_6 signature with key measurements of bacterial load, such as baseline MGIT time to detection (TTD) and sputum Ziehl-Neelsen scores, were explored. However, the MAMS_6 score did not show significant correlations with log mean TTD values or sputum Ziehl-Neelsen scores at baseline, nor with fold changes between Week 0 and week 2.

In summary, while differential regulation of several genes over the TB treatment course varied between fast and slow culture converters, the magnitude in LogFold change (LFC) was minimal. Nevertheless, the MAMS_6 signature demonstrated significant correlation with the time surrounding culture conversion, underscoring its potential utility in clinical settings.

### Example 7: MAMS_6 transcriptomic signature holds diagnostic potential

To test whether our 6-gene signature for cure could also be employed for pulmonary and extrapulmonary TB diagnostic purposes, we evaluated the expression of the 6 genes in publicly available transcriptomic datasets representing more heterogenous patient groups. To this end, mean normalised transcript levels for all 6 genes were calculated per sequenced patient. Further, signature scores for two other parsimonious TB diagnostic signatures (Sweeney_3 and RISK6) were calculated for comparison.

ROC curve analyses between different patient groups showed comparable results for all 3 signatures in the different cohorts (Figure 10). Distinction between healthy controls (n=50 IGRA negative contacts) (Figure 10A) or TB infection (TBI, n= 49 IGRA positive contacts) (Figure 10E) and culture confirmed Tuberculosis disease (n=53) in the RNA sequencing datasets of the Leicester, UK cohort (GSE107994), enrolling adults testing negative for HIV, is comparatively good for all 3 parsimonious signatures (AUC healthy vs TB: MAMS_6 0.986, RISK6 0.991, Sweeney_3 0.978; TBI vs TB: MAMS_6 0.965, RISK6 0.971, Sweeney_3 0.951). Here, all 3 reached the minimal WHO TPP triage requirements of 90% sensitivity and 70% specificity. Correct classification of culture confirmed tuberculosis disease (n = 194) from TBI (n = 167) or various other diseases (OD) including respiratory diseases (n = 175) in two African cohorts analysed by microarray (GSE37250) decreased for all 3 tested signatures (AUC TBI vs TB: MAMS_6 0.898, RISK6 0.769, Sweeney_3 0.856; OD vs TB: MAMS_6 0.750, RISK6 0.832, Sweeney_3 0.806) (Figure 10 B/F), the MAMS_6 signature achieving highest performance in classifying TBI from TB. In the classification of paediatric patients in an Indian cohort with TB (n= 35) from TBI (n=68) or other diseases (n=224) the signatures show a broader range (AUC TBI vs TB: MAMS_6 0.903, RISK6 0.740, Sweeney_3 0.974; OD vs TB: MAMS_6 0.792, RISK6 0.763, Sweeney_3 0.936) (Figure 10 C/G). All signatures also show good performance in the classification from extrapulmonary TB (n=47) vs controls (n=61) (AUC control vs extrapulmonary TB: MAMS_6 0.962, RISK6 0.956, Sweeney_3 0.956) (Figure 10 D).

Splitting the participants by HIV status, shows decreased performance of all 3 signatures for adults living with HIV (PLH) in distinguishing TBI from clinical TB disease (Figure 11 A). Similarly, performance of MAMS_6 and Sweeney_3 also decreased in PLH for the distinction of OD from TB disease (Figure 11 B). In the paediatric cohort only 10 children were living with HIV precluding definitive conclusions on signature performance in children living with HIV (Figure 11C).

### Example 8: MAMS_6 transcriptomic signature predicts recurrence of TB

ROC curve analyses of patients with recurrent disease from those without, shows MAMS6 has good performance in predicting of relapses at EOT (Figure 12).

Altogether, these analyses showed that the new MAMS_6 signature holds diagnostic potential, comparable to previously published signatures.

## Claims

1. **A method for the diagnosis, prognosis, stratification and/or monitoring of an infectious disease,** in a subject, comprising the steps of:
(a) Providing a biological sample from the subject,
(b) Determining the level of at least two or more biomarker(s) selected from the group consisting of AIM2, GBP1, FCGR1B, CD274, SMARCD3, and FLVCR2, in the biological sample,
wherein a differential level of at least one biomarker(s) in the biological sample from the subject as determined in step (b) compared to a healthy control or reference value is indicative for the presence of the infectious disease in the subject.

2. The method of claim 1, wherein step (b) comprises determining the level of at least two or more of the biomarker(s) in the biological sample, preferably determining the level of at least four or more of the biomarker(s) in the biological sample, more preferably determining the level of at least six or more of the biomarker(s) in the biological sample.

3. The method of claim 1 or 2, wherein the method comprises further steps
(c) of determining the level of at least one, preferably of at least two, most preferably at least three, control-marker(s) in the biological sample, wherein the control-marker is not a biomarker, preferably wherein the at least one control-marker is a housekeeping control-marker, more preferably wherein the at least one control-marker is selected from a group comprising B2M, HPRT1 and VIM;
(d) normalizing the level of the at least two or more biomarker(s) as determined in (b) with the level of the at least one control-marker as determined in (c).

4. The method of any one of claims 1 to 3, wherein step (b) comprises determining the level of at least AIM2, and GBP1, in the biological sample.

5. The method of any one of claims 1 to 4, wherein step (b) comprises determining the level of at least AIM2, FCGR1B, CD274, and GBP1, in the biological sample.

6. The method of any one of claims 1 to 5, wherein step (b) comprises determining the level of at least AIM2, FCGR1B, CD274, GBP1, SMARCD3, and FLVCR2, in the biological sample.

7. The method of any one of claims 1 to 6, wherein the biological sample is a tissue sample or body liquid sample, preferably a blood sample, most preferably a whole blood sample.

8. The method of any one of any of claims 1 to 7, wherein the biomarker is an RNA, such as an mRNA transcript, biomarker.

9. The method of any one of any of claims 1 to 8, wherein the subject is a human, such as an adult human, for example aged 18 to 90, or is a child or adolescent human for example aged 0 to 17.

10. The method of any one of any of claims 1 to 9, wherein the infectious disease is a bacterial disease, such as tuberculosis, for example caused by an infection with bacteria of the Mycobacterium tuberculosis complex, preferably caused by *Mycobacterium tuberculosis .*

11. The method of any one of the preceding claims, wherein the biomarker is detected using one or more method for the quantification of transcript levels in a sample, such as quantitative Polymerase Chain Reaction (qPCR), Loop-mediated Isothermal Amplification (LAMP), next generation sequencing (NGS), probe-based nucleic acid hybridization assays (such as Northern Blots and similar technologies).

12. The method of any one of the preceding claims, wherein the method is for the monitoring of a therapy, in particular therapy success, in the subject, and/or wherein the method is a screening method.

13. The method of any one of the preceding claims, wherein the method is for distinguishing between a first infectious disease caused by an infection with Mycobacterium tuberculosis and a second disease, preferably wherein the second disease is sarcoidosis,
and/or wherein the method is for the detecting a latent or active infection causing the infectious disease, preferably is a tuberculosis infection (latent tuberculosis infection) or is a clinical tuberculosis disease (active tuberculosis infection), preferably wherein the at least one biomarker is a panel of AIM2, FCGR1B, CD274, GBP1, SMARCD3, and FLVCR2.

14. **A diagnostic kit** for performing a method of any one of the preceding claims, preferably comprising one or more means for the detection of the at least one biomarker(s), preferably wherein the means are selected from a nucleic acid primer and/or probe, or is an antibody.

15. **Use of a nucleic acid probe or nucleic acid primer in a diagnosis, prognosis, stratification and/or monitoring of an infectious disease,** wherein such nucleic acid probe or nucleic acid primer hybridizes to a transcript of at least one biomarker selected from the group consisting of AIM2, GBP1, FCGR1B, CD274, SMARCD3, and FLVCR2, and optionally wherein the nucleic acid primer hybridizes to a transcript of one or more control-marker, preferably wherein the control-marker is selected from the group comprising B2M, HPRT1, and VIM, preferably wherein the use is in a method of any one of claims 1 to 13.
